# EUROPEAN PATENT SPECIFICATION

(11) **EP 2 057 934 B1**
(45) Date of publication and mention of the grant of the patent: **04.01.2017**
(21) Application number: 07120485.3
(22) Date of filing: 12.11.2007
(51) Int. Cl.: A61B 1/04, A61B 1/273, A61B 5/00, A61B 5/07

(54) **Device for hemorrhage detection**
Vorrichtung zum Erkennen von Blutungen
Dispositif de détection d'hémorragie

(43) Date of publication of application: 13.05.2009
(73) Proprietor: NOVINEON HEALTHCARE TECHNOLOGY PARTNERS GMBH, 72074 Tübingen (DE)
(72) Inventor: Schostek, Sebastian, 72070, Tübingen (DE); Rieber, Fabian, 70193, Stuttgart (DE); Ho, Chi-Nghia, 70180 Stuttgart (DE); Schurr, Marc Oliver, Prof. Dr. med, 72072, Tübingen (DE)
(74) Representative: Winter, Brandl, Fürniss, Hübner, Röss, Kaiser, Polte - Partnerschaft mbB

(56) References cited:
- EP-A- 1 875 858
- WO-A-02/073507
- WO-A-2006/005075
- US-A1- 2005 154 277

## Description

The present invention relates to a device for hemorrhage detection.

Bleedings in the digestive tract may occur due to different health disturbances of the digestive tract, e.g. esophageal varices or gastric or duodenal ulcers. Even after an endoscopic treatment of such disturbances of health, there often occur recurrent bleedings that constitute a severe complication. Recurrent bleedings may occur several hours or days after a treatment, which renders their detection difficult. Bleedings in the digestive tract may lead to an acute emergency situation, since they may entail a large loss of blood if they go unnoticed. Therefore it is necessary to reliably and early detect such bleedings.

US 2005/0154277 A1 discloses a capsule for the absorption spectroscopy diagnosis so as to compare the ratio of oxyhemoglobin and de-oxyhemoglobin concentrations by using two LEDs, one of which emits light with a wavelength of 660nm and the other of which emits light with a wavelength of 940nm.

It is an object of the present invention to provide an improved device for hemorrhage detection, said device using no colorants, being easy in application and reliably detecting bleedings.

It is a further object of the present invention to provide a device for hemorrhage detection, said device being adapted to ensure a continuous monitoring of lesions bearing a risk of bleeding.

The objects of the invention are achieved with a device for hemorrhage detection according to claim 1.

Further advantageous developments of the invention are subject-matter of the dependent claims.

According to the invention, the device for hemorrhage detection consists of a capsule and at least one detecting means provided inside the capsule for detecting a presence or non-presence of blood, wherein the capsule has an outer shape and a dimension to be swallowable by a patient. I.e. the capsule is designed such that it can be swallowed by a patient on his own without any auxiliary means.

Accordingly, for detection of a bleeding e.g. in the digestive tract, the above described capsule is simply swallowed by a patient and naturally reaches the digestive tract, where the detecting means detects the presence or non-presence of blood. After that the capsule is naturally excreted from the patient's body through the digestive tract.

Preferably, the device for hemorrhage detection further comprises a sending means for sending the detection result of the detecting means, i.e. whether or not blood is present, the sending means being provided inside the capsule. Preferably, the device for hemorrhage detection also comprises a receiving means for receiving the sent by sending means. The receiving means is preferably located outside a patient's body, i.e. extracorporeal. The transmission of data from the sending means to the receiving means is effected by wireless transmission. The device for hemorrhage detection designed as described above can be advantageously applied for an immediate diagnosis or in an emergency case, where e.g. a detection of blood by means of an endoscope is not possible.

Alternatively, the device for hemorrhage detection may be equipped with a storage device for storing detection results of the detecting means, which are read after the device for hemorrhage detection has been excreted from the patient's body through the digestive tract.

Preferably, an outer shape of the capsule is in a rounded shape for improving the swallowability by a patient. Possible shapes are e.g. a pill-like or tablet-like shape, a spherical shape or a cylindrical shape with rounded edges/ends.

Preferably, the detecting means detects a presence or non-presence of blood in a substance/content by measuring specific absorption spectra of the substance/content.

According to the invention, the detecting means comprises a light source, preferably at least one LED, and a photosensitive sensor, preferably a photodiode or a phototransistor, for receiving light emitted from the light source.

For measuring the presence or non-presence of blood, the light source emits light of at least two different wavelengths being absorbed to a different degree by blood or blood components. Especially, the light source emits light in the violet range of the visible spectrum of about 380-450nm, and light in the red range of the visible spectrum of about 620-750nm.

According to the invention, the capsule is provided with at least one recess or groove, wherein the light source and the photosensitive sensor are arranged such that the light emitted by the light source passes the recess or groove such that the light can be at least partly detected by the photosensitive sensor. I.e. the light emitted by the light source leaves the capsule within or near the recess or groove, passes the recess or groove outside of the capsule, and enters the capsule again within or near the recess or groove in a way that it can at least partly be detected by the photosensitive sensor.

According to the invention, the light source and the photosensitive sensor are arranged such that they face each other (are arranged opposed to each other) via the recess or groove.

According to the invention, the recess or groove extends in a direction perpendicular to a longitudinal axis of the capsule.

According to the invention, the device for hemorrhage detection determines a presence or non-presence of blood by comparing the intensities of the light of the at least two different wavelengths emitted from the light source and detected by the photosensitive sensor.

Optionally the device for hemorrhage detection may be adapted to differentiate between different manifestations of blood, e.g. whether the blood is venous, arterial, or clotted blood. This can be realized by e.g. (additionally) emitting infrared light from the light source.

Preferably, the device for hemorrhage detection further comprises a photo/video camera, preferably in form of a CCD-sensor, provided in the capsule for capturing images of the vicinity of the capsule.

In the following, the invention shall be described in greater detail by means of embodiments with reference to the accompanying drawings.
Fig. 1 is a schematic view of a device for hemorrhage detection according to an embodiment of the invention.
Fig. 2 is a schematic view of a device for hemorrhage detection according to a modification of the embodiment of the invention.
Fig. 3 is a schematic view a detection means of a device for hemorrhage detection according to a non-claimed embodiment.
Fig. 4 is a schematic partial view of a device for hemorrhage detection according to a modification of the non-claimed embodiment.
Fig. 5 is a schematic partial view of a device for hemorrhage detection according to a non-claimed modification of the embodiment of the invention.

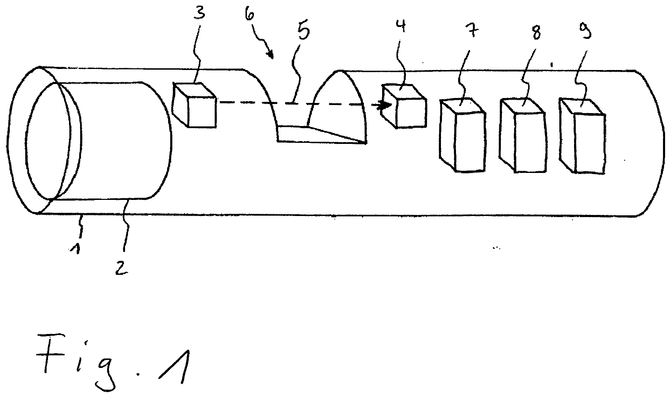

Fig. 1 shows a device for hemorrhage detection (referred to as "device" below) according to an embodiment of the invention. The device basically consists of a capsule 1 and a detecting means provided inside the capsule 1 for detecting presence or non-presence of blood.

The capsule 1 has an outer shape and a dimension such that it is swallowable by a human-being. I.e. the capsule can be swallowed by a patient himself without using any further auxiliary means. Although not shown in detail in Fig. 1, the capsule 1 preferably consists of a cylindrical body, wherein opposite ends of the cylindrical body are respectively formed in a semi-sphere shape for improving the swallowability of the capsule 1. Alternatively, only the edges of the cylindrical body may be rounded. Further preferred outer shapes of the capsule are a pill-like shape, a tablet-like shape, an oval shape or an egg shape. The outer shape of the capsule 1 is not limited to the above shapes, but can be any shape facilitating a swallowing of the capsule 1 by a human-being. Preferably, the capsule has a length of about 25-30 mm and a diameter or maximum width of about 5-6 mm. Advantageously, the capsule 1 is made of a biocompatible material, e.g. a biocompatible plastic material.

The detecting means basically consists of a light source 3 and a photosensitive sensor 4. The detecting means detects the presence or non-presence of blood in a substance or content by measuring specific absorption spectra of the substance or content. E.g., due to the hemoglobin, blood absorbs light in the violet range of the visible spectrum, i.e. light of a wavelength of about 380-450 nm, to a higher degree than light in the red range of the visible spectrum, i.e. light of a wavelength of about 620-750 nm. Accordingly, it is possible to determine the presence or non-presence of blood in a substance or content e.g. by measuring and comparing the transmissibility of light in the violet range and the transmissibility of light in the red range of the visible spectrum through the substance or content.

The light source 3 emits alternately the light of different wavelengths, i.e. light in the violet range of visible spectrum (about 415nm) and light in the red range of the visible spectrum (about 700nm). The emitting of the light of different wavelengths can be performed successively or in a pulsed manner. Preferably, the light source 3 consists of two LEDs, wherein one emits light in the violet range and the other one emits light in the red range of the visible spectrum. However, the invention is not limited to such a construction and it is possible to provide only one LED which is able to emit light both in the violet range and in the red range of the visible spectrum. The photosensitive sensor 4 is adapted to receive the light emitted by the light source 4 and is preferably a photodiode or a phototransistor. Preferably the photosensitive sensor 4 consists of one photodiode or phototransistor adapted to detect the intensities of both light in the violet range and light in the red range of the visible spectrum. However, the invention is not limited to this and the photosensitive sensor 4 may consist of two photodiodes or phototransistors, with one of them detecting the intensity of light in the violet range and the other one of them detecting the intensity of light in the red range of the visible spectrum.

As shown in Fig. 1, the light source 3 and the photosensitive sensor 4 are arranged such that they are opposed to each other (face each other) via a recess 6 formed in the capsule 1. In particular, the light source 3 and the photosensitive sensor 4 are arranged such that light 5 emitted from the light source 3 passes the recess 6 and is received by photosensitive sensor 4. The recess 6 is preferably a cutout or groove. The edges of the recess 6 are rounded so as to improve the swallowability of the capsule 1. The recess 6 forms a free space into which a substance/content to be examined can be introduced (into which a substance/content to be examined can flow) for measuring whether the substance/content contains blood or not. I.e. the recess 6 forms a measuring/examination area of the device. The recess extends perpendicular to the longitudinal axis of the capsule 1.

The device further comprises an energy source 2, e.g. a battery, for supplying the respective components of the device with energy, a processing means 7, an analog-digital converter 8 and a sending means 9, all of which are provided inside the capsule 1.

The processing means 7, e.g. a CPU, controls the detecting means, i.e. the light source 3 and the photosensitive sensor 4, the analog-digital converter 8 and the sending means 9, and optionally evaluates and stores the data received by the photosensitive sensor 4. Optionally, the processing means 7 also comprises analog signal processing means, e.g. an analog signal amplifier to amplify the analog sensor signal of the photosensitive sensor 4. The analog-digital converter 8 converts the analog signals obtained from the detecting means into digital signals, and the sending means 9 sends the data evaluated/processed by the processing means 7 to an extracorporeal part (not shown). The data sent by the sending means 9 may e.g. be measured values, status information, or signals indicating an occurrence or non-occurrence of a bleeding (presence of non-presence of blood within the recess).

The extracorporeal part is basically composed of a receiving means, an interface, and an information output means. The receiving means receives data sent by the sending means 9 via a wireless transmission. The information output means connected to the receiving means via the interface presents the data received from the receiving means to a user, e.g. a nurse or a medical doctor. The information output means may e.g. be an optical device or an acoustic signaling device, such as a display or a loudspeaker. Furthermore, the data received by the receiving means may be transmitted by means of the interface to third parties, e.g. a medical doctor or an emergency hotline/center. Such a transmission may be realized by the use of e.g. DECT, Bluetooth, WLAN, GSM or satellite.

Optionally, the device may further comprise a video camera or a photo camera (not shown) provided inside the capsule 1 for capturing images in the vicinity of the capsule 1. With such a camera blood in a substance/or content, a bleeding source itself or an ulcer, for example, can be visually detected. Preferably, the camera is provided in form of a CCD-sensor. The image information received from the camera can be sent via the sending means 9 to the extracorporeal part similarly to the information of the detecting means, and can be put out by the information output means, so that a user, e.g. medical staff, can see the images captured by the camera. Alternatively, the captured images may be stored in an internal storing means and read by means of a reading device after the device has been naturally excreted through the digestive tract.

Hereinafter, the function of the device according to the embodiment of the invention shall now be described under consideration of an example of detecting blood in the digestive tract of a patient.

In order to reach the location where the presence or non-presence of blood shall be detected, the device is simply swallowed by the patient and naturally reaches after a certain time the digestive tract. Accordingly for bringing the device to the desired location for detection, no further technical means are necessary. When the device has reached the desired location for detection, the device measures whether blood is contained in the content of the digestive tract. For example, the beginning of the measurement can be started automatically after a predetermined time, upon activating the device before swallowing (i.e. in advance), by activating the device by means of a magnetic reed switch or a signal (e.g. received by the sending means 9, which then also has a function of a receiver), etc. The detection whether blood is contained in the digestive tract functions as follows:

The light source 3 emits alternately and successively light in the violet range and light in the red range of the visible spectrum upon command of the processing means 7 (the sending frequency may be variably set, preferably every 1 or 5 seconds, i.e. 0.2-1 Hz). The respective emitted light 5 travels through the recess 6 which is filled with a part of the content of the digestive tract, in particular with a part of the liquid present in the digestive tract. While traveling through the recess 6, the respective emitted light 5 is absorbed in dependency of the composition of the content of the digestive tract. The photosensitive sensor 4 then detects the respective light 5 which has not been absorbed and produces a signal on the basis of each respective light 5 detected.

As described above, blood has a low absorption rate for light in the red range of the visible spectrum, but a high absorption rate for light of violet range of the visible spectrum (i.e. the transmissibility for light in the red range of the visible spectrum is higher than the transmissibility for light of the violet range of the visible spectrum). Therefore, if the content present in the recess 6 contains blood, the light in the red range of the visible spectrum is absorbed to a minor degree than light in the violet range of the visible spectrum. As a result, the quantity/intensity of light of the violet range of the visible spectrum received by the photosensitive sensor 4 is reduced relatively to the quantity/intensity of light in red range of the visible spectrum received by the photosensitive sensor 4. Thus, by comparing the quantities/intensities of the light of the red range of the visible spectrum received by the photosensitive sensor 4 and the quantity/intensity of light in the violet range of the visible spectrum received by the photosensitive sensor 4 it is possible to determine whether blood is present or not present in the content of the digestive tract.

In the present embodiment such a comparison is made by the processing means 7 which receives the respective intensities/quantities detected by the photosensitive sensor 4 in the form of a signal converted via the A/D-converter 8. The signal is evaluated and processed by the processing means 7. The thus obtained information is then sent via the sending means 9 to the receiving means of the extracorporeal part being located outside of the patient's body. The information received by the receiving means is then outputted from the information output means, such as a screen or a loudspeaker, so that a user, e.g. a doctor, can notice whether blood is present in the digestive tract of the patient or not.

Preferably, the comparison of the quantities/intensities of the light if the red range and the light of the violet range of the visible spectrum is made by calculating the ratio of the quantities/intensities of the red and violet light, preferably by dividing the quantity/intensity of the transmitted red light by the quantity/intensity of the transmitted violet light. Thus, in case the violet light is absorbed by the recess content to a higher degree compared to the red light, and thus the sensor signal of measuring the transmitted red light is higher than the sensor signal of measuring the transmitted violet light, the ratio will increase. In case of blood being present within the recess, the ratio will increase above a threshold, so that the processing means 7 will be able to determine the presence of blood. To inform the user about this event immediately, the processing means 7 can transfer via the sensing means 9 a respective event signal to the extracorporeal receiving means.

### [Modifications]

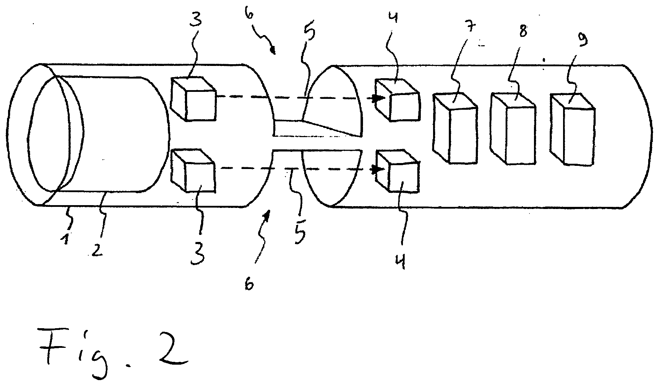

Fig. 2 shows a modification of the embodiment of the invention. The device shown in Fig. 2 is the same as that according to the embodiment, except that the capsule 1 is provided with a second detecting means (a second light source 3 and a second photosensitive sensor 4) and a corresponding second recess 6. The second detecting means and the second recess 6 are designed in the same manner as described above. The detecting means and the recesses, respectively, are arranged diametrically opposed to each other with respect to an axial direction of the capsule 1. In detail, the detecting means and the recesses, respectively, are symmetrically arranged with respect to a plane including the longitudinal axis of the capsule. However, the second detecting means and the recesses, respectively, need not to be arranged as described above and can be positioned arbitrary. The provision of the second detecting means improves the detection accuracy/range of the device. Apart from that, further detection means may be provided.

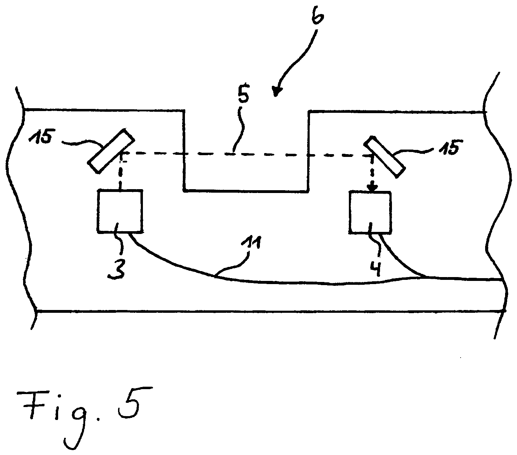

Fig. 5 shows a further non-claimed modification of the embodiment of the invention. The device shown in Fig. 5 is the same as according to the embodiment of the invention, except the capsule 1 is provided with reflecting means 15. This reflecting means 15 consists of one or more light-reflecting components, such as mirrors or objects coated with materials having light-reflecting properties such as chrome. In case the light emitting area of the light source 3 is not directly facing the photosensitive area of the photosensitive sensor 4 via the recess 6, the light can be directed / guided by the reflecting means 15 in a way that the light 5 emitted by the light source 3 travels through the recess and further is led to the photosensitive sensor. The light-reflecting components may have a flat surface or a domed surface, such as a convex or concave surface, to focus or disperse the light.

Other components, features and the function of the device are the same like that of the embodiment.

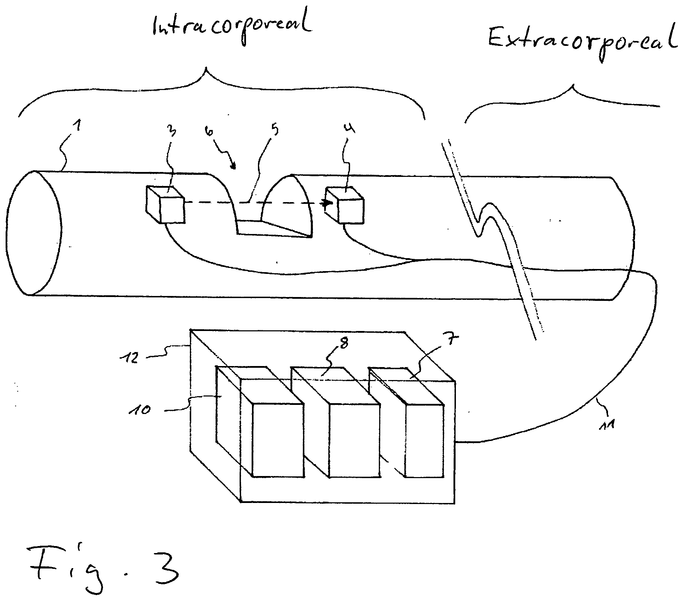

Fig. 3 shows a detecting means of a device for hemorrhage detection (below also referred to as "device") according to a non-claimed embodiment.

The device according to this embodiment basically consists of a catheter (not shown) and a detecting means secured to an end of the catheter. The detecting means is shown in Fig. 3.

The detecting means is the same as in the above described embodiment of the invention and basically consists of a light source 3 and a photosensitive sensor 4. The detecting means detects the presence or non-presence of blood in a substance or content by measuring specific absorption spectra of the substance or content.

The light source 3 emits alternately the light of different wavelengths, preferably light in the violet range of the visible spectrum (about 380-450nm) and light in the red range of the visible spectrum (about 620-750nm). The emitting of the light of different wavelengths can be performed successively or in a pulsed manner. Preferably, the light source 3 consists of two LEDs, wherein one emits light in the violet range and the other one emits light in the red range of the visible spectrum. However, the invention is not limited to such a construction and it is possible to provide only one LED which is able to emit light both in the violet range and in the red range of the visible spectrum. The photosensitive sensor 4 is adapted to receive the light emitted by the light source 4 and is preferably a photodiode or a phototransistor. Preferably the photosensitive sensor 4 consists of one photodiode or phototransistor adapted to detect the intensities of both light in the violet range and light in the red range of the visible spectrum. However, the invention is not limited to this and the photosensitive sensor 4 may consist of two photodiodes or phototransistors, with one of them detecting the intensity of light in the violet range and the other one of them detecting the intensity of light in the red range of the visible spectrum.

As shown in Fig. 3, the detecting means is provided inside a capsule 1. Preferably, the capsule 1 consists of a cylindrical body, wherein opposite ends of the cylindrical body are formed in a semi-sphere shape. Alternatively, only the edges of the cylindrical body are rounded. Further preferred outer shapes of the capsule are a pill-like shape, a tablet-like shape, an oval shape, egg-shape or a spherical shape. The outer shape of the capsule 1 is not limited to the above shapes. Preferably, the capsule has a length of about 25-30 mm and a diameter or maximum width of about 5-6 mm. Advantageously, the capsule 1 is made of a biocompatible material, e.g. a biocompatible plastic material.

As shown in Fig. 3, the light source 3 and the photosensitive sensor 4 are arranged such that they are opposed (face toward each other) to each other via a recess 6 formed in the capsule 1. In particular, the light source 3 and the photosensitive sensor 4 are arranged such that the light 5 emitted from the light source 3 passes the recess 6 and is received by the photosensitive sensor 4. The recess 6 is preferably a cutout or groove formed in the capsule 1, but can have another shape, e.g. the recess can also be a hole. Preferably the edges of the recess 6 are rounded. The recess 6 forms a free space into which a substance/content to be examined can be introduced (into which a substance/content to be examined can flow) for measuring whether the substance/content contains blood or not. I.e. the recess 6 forms a measuring/examination area of the device. The recess extends perpendicular to the longitudinal axis of the capsule 1.

Optionally, the device may additionally be provided with a photo/video camera, preferably a CCD-Sensor, provided in the capsule 1 for capturing images in the vicinity of the capsule 1 and catheter, respectively, as described in the embodiment of the invention.

The detecting means is secured via the capsule 1 to an end of the catheter (in this context catheter means any pipe shaped device which is to be inserted into a human body through an artificial (man-made) aditus). The capsule 1 comprising the detecting means is arranged so as not to affect (or to affect as minimal as possible) the function of the catheter, e.g. the draining of body fluid from a patient, and so as not to affect the function of the detecting means, i.e. the detecting means are arranged such that a substance/content to be measured easily reaches the recess 6.

Preferably, the detecting means are arranged such that at least the portion of the capsule 1 where the recess 6 is formed protrudes from the end of the catheter. However, the detecting means also may be arranged completely inside the catheter. The detecting means may be secured to an inner face and/or an outer surface and/or to the end surface of the catheter. The detecting means may be secured the catheter e.g. by a clip connection enabling that the capsule 1 can be clipped onto the catheter, inserting a portion of the capsule 1 in a recess formed in the catheter, adhering the capsule 1 to the catheter, a stick connection, etc.

The device further comprises an output means 12 connected to the detecting means via an information transferring element 11. The output means 12 comprises at least an processing means 7 processing and/or evaluating information received from the detecting means via the information transferring element 11 and controlling the detecting means. Preferably, the output means 12 further comprises an A/D-converter and/or an information output means 10, e.g. a display or a loudspeaker. In contrast to the detecting means, the output means 12 are not intended to be inserted into a patient's body but are intended to remain outside a patient's body, i.e. extracorporeal. Preferably, the output means 12 are provided near an end of the catheter which is opposite to the end at which the detecting means is secured. The output means 12 or a portion of the output means 12 may be secured to the catheter, e.g. by adhering or clipping, or may be completely or partially formed integrally with the catheter. However, this is no necessity. The output means 12 may further comprise an interface enabling to transmit detected information to third parties, e.g. a doctor or an emergency hotline/center, e.g. via DECT, Bluetooth, WLAN, GSM or satellite.

The information transferring element 11 transfers information/data from the detecting means to the output means 12 and vice versa (e.g. signals for operating the detecting means). The information transferring element 11 extending through the capsule 1 may be guided along an interior surface or an exterior surface of the catheter from the detecting means to the output means 12. It may also be possible to guide the information transferring element 11 at least partially within a wall forming the catheter. Further, the information transferring element 11 may be formed integrally with the catheter.

The function of the device according to this embodiment is as follows.

The catheter together with the detecting means secured at an end thereof are inserted through an artificial/hand-made aditus into a human body. The catheter is inserted such that the detecting means is located in the intended position for detection e.g. in the interior of a hollow organ such as the digestive tract which is taken as an example in the following. Accordingly, for bringing the device to the desired location for detection, no further technical means are necessary. When the device has reached the desired location for detection, the device measures whether blood is contained in the content of the digestive tract. The beginning of the measurement can e.g. be started by sending a signal to the processing means 7. The detection whether blood is contained in the digestive tract functions in the same way as in the embodiment of the invention and is as follows:

The light source 3 emits alternately and successively light in the violet range and light in the red range of the visible spectrum upon command of the processing means 7 (the sending frequency may be variably set, preferably 1 or 5 seconds). The respective emitted light 5 travels through the recess 6 which is filled with a part of the content of the digestive tract, in particular with a part of the liquid present in the digestive tract. While traveling through the recess 6, the respective emitted light 5 is absorbed in dependency of the composition of the content of the digestive tract. The photosensitive sensor 4 then detects the respective light 5 which has been absorbed and produces a signal on the basis of each respective light 5 detected.

As described above, blood has a low absorption rate for light in the red range of the visible spectrum, but a high absorption rate for light of the violet range of the visible spectrum (i.e. the transmissibility for light in the red range of the visible spectrum is higher than the transmissibility for light in the violet range). Therefore, if the content present in the recess 6 contains blood, the light in the red range of the visible spectrum is absorbed to a minor degree than the violet light. As a result, the quantity/intensity of light of the red range of the visible spectrum received by the photosensitive sensor 4 is reduced relatively to the quantity/intensity of violet light received by the photosensitive sensor 4. Thus, by comparing the quantities/intensities of the light of the red range of the visible spectrum received by the photosensitive sensor 4 and the quantity/intensity of violet light received by the photosensitive sensor 4 it is possible to determine whether blood is present or not present in the content of the digestive tract.

In the present embodiment such a comparison is made by the processing means 7 which receives the respective intensities/quantities detected by the photosensitive sensor 4 via the information transferring element 11 in the form of a signal converted via the A/D-converter 8. The signal is evaluated and processed by the processing means 7. The thus obtained information is then outputted via the information output means 10, e.g. a screen, so that a user, e.g. a doctor, can notice whether blood is present in the digestive tract of the patient or not.

### [Modification]

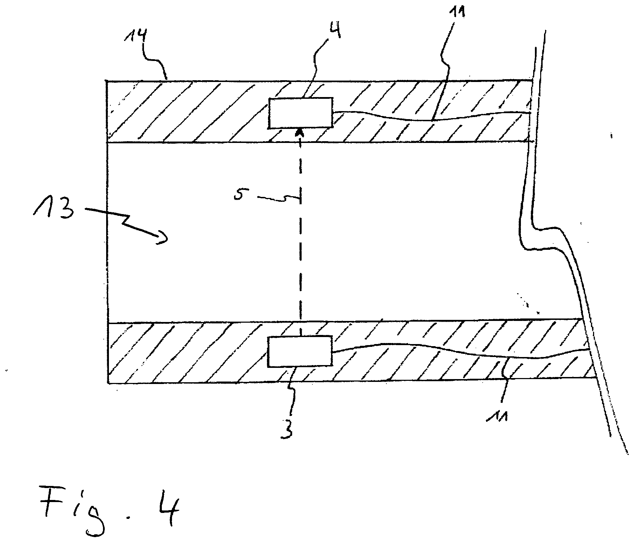

Fig. 4 shows a modification of the non-claimed embodiment. The modification of the non-claimed embodiment differs from the non-claimed embodiment in that the detecting means is not secured to the catheter via a capsule, but is integrally formed with the catheter.

As can be seen from Fig. 4, the light source 3 and the photosensitive sensor 4 constituting the detecting means are formed integrally with the catheter 14 at an end thereof. The light source 3 and the photosensitive sensor 4 may be provided completely within a wall forming the catheter 14, so as to be encapsulated by the catheter itself. Alternatively, the light source 3 and the photosensitive sensor 4, respectively, may be provided in a recess formed in an inner surface of the catheter 14 or may be directly fixed to the inner surface of the catheter, e.g. by means of an adhesive. Preferably, the light source 3 and the photosensitive sensor 4 are arranged such that they face each other via an interior 13 of the catheter. More preferably, they are arranged diametrically opposed to each other with respect to an axial direction of the catheter 14. In the device of the modification of the non-claimed embodiment, the interior 13 of the catheter 14 corresponds to the recess 6 of the device of the non-claimed embodiment. I.e. the light emitted by the light source 3 passes the interior 13 and is received by the photosensitive sensor 4; on basis of the light received by the photosensitive sensor 4 it is then judged whether a substance/content present in the interior 13 of the catheter contains blood or not.

As can be seen further from Fig. 4, two information transferring elements 11 are provided connecting the light source 3 and the photosensitive sensor 4, respectively, to output means (being identical to the output means 12 of the non-claimed embodiment) for transferring data from the detecting means to the output means and vice versa.

Preferably the information transferring elements 11 are guided at least partially within the wall forming the catheter 14, as can be seen from Fig. 4. However, the information transferring elements 11 also may be guided along an interior surface or an exterior surface of the catheter 14. Further, the information transferring element 11 may be formed integrally with the catheter.

Other components, features and the function of the device according to the modification of the non-claimed embodiment are the same as in the non-claimed embodiment.

Further, it may also be possible to judge whether a specific component or element other than blood is present in the substance/content to be examined. For this purpose, the light source may be adapted to emit light of (a) further different wavelength(s) showing a specific absorption characteristic with respect to the specific component or element which shall be detected. The photosensitive sensor has to be adapted correspondingly. Moreover, the device for hemorrhage detection according to the invention may be designed to detect different manifestations of blood. Different manifestations of blood are e.g. clotted blood, venous blood, or arterial blood. For this purpose, the light source may e.g. be adapted to also emit infrared light, as the infrared light is absorbed to a different degree in dependency of the manifestation of blood. The photosensitive sensor has to be adapted correspondingly.

## Claims

1. A device for hemorrhage detection consisting of a capsule (1) and at least one detecting means (3, 4) provided in the capsule (1) for detecting a presence or non-presence of blood,
wherein the capsule (1) has an outer shape and a dimension to be swallowable by a patient,
wherein the detecting means comprises a light source (3), preferably at least one LED, and a photosensitive sensor (4), preferably at least one photodiode or phototransistor, for receiving light emitted from the light source (3),
wherein the light source (3) is adapted to alternately emit light of at least two different wavelengths being absorbed to a different degree by blood or blood components, namely light in the violet range of the visible spectrum having a wavelength in the range of 380-450nm and light in the red range of the visible spectrum having a wavelength in the range of 620-750 nm,
wherein the photosensitive sensor is adapted to detect the light at the at least two wavelengths that has not been absorbed by blood or blood components, and
wherein the device is adapted to determine a presence or non-presence of blood by comparing the intensities of the light of the at least two different wavelenghts detected by the photo-sensitive sensor,
wherein at least one recess (6) is formed in the capsule (1), extends in a direction perpendicular to a longitudinal axis of the capsule and has edges being rounded to improve the swallowability of the capsule (1),
wherein the light source (3) and the photosensitive sensor (4) are arranged such that they face each other via the at least one recess (6), and
wherein the at least one recess (6) forms a free space into which a substance/content to be examined can flow.

2. The device for hemorrhage detection according to claim 1, wherein the outer shape of the capsule (1) is a pill-like shape, an oval shape, an egg shape or a cylindrical shape with rounded edges/ends.

3. The device for hemorrhage detection according to claim 1 or 2, wherein the at least one detecting means (3, 4) is adapted to detect a presence or non-presence of blood in a substance/content by measuring specific absorption spectra of the substance/content.

4. The device for hemorrhage detection according to claim 1, wherein the light source (3) and the photosensitive sensor (4) are arranged such that the light (5) emitted by the light source (3) passes the recess (6) and can at least partly be detected by the photosensitive sensor (4).

5. The device for hemorrhage detection according to any one of the above claims, wherein the device for hemorrhage detection is further adapted to differentiate between different manifestations of blood, e.g. whether the blood is venous, arterial, or clotted blood.

6. The device for hemorrhage detection according to any one of the above claims, further comprising a photo/video camera, preferably in form of a CCD-sensor, provided in the capsule (1) for capturing images of the vicinity of the capsule (1).

7. The device for hemorrhage detection according to claim 1, further comprising a sending means (9) provided inside the capsule (1) for sending data detected by the detecting means (3, 4).

## Patentansprüche

1. Ein Gerät zur Blutungserkennung, mit einer Kapsel (1) und mindestens einem Erkennungsmittel (3, 4), welches in der Kapsel (1) vorgesehen ist, um die An- oder Abwesenheit von Blut zu erkennen,
wobei die Kapsel (1) eine solche äußere Form und Abmessungen aufweist, um von einem Patienten geschluckt werden zu können,
wobei die Erkennungsmittel eine Lichtquelle (3), vorzugsweise zumindest eine LED, und einen photosensitiven Sensor (4) umfasst, vorzugsweise mindestens eine Photodiode oder Phototransistor, zum Empfang von Licht, welches von der Lichtquelle (3) ausgesendet wurde,
wobei die Lichtquelle (3) dazu angepasst ist, abwechselnd Licht mindestens zweier verschiedener Wellenlängen zu emittieren, welches zu einem unterschiedlichen Grad durch Blut oder Blutkomponenten absorbiert wird, nämlich Licht im violetten Bereich des sichtbaren Spektrums mit einer Wellenlänge von 380-450nm, und Licht im roten Bereich des sichtbaren Spektrums mit einer Wellenlänge im Bereich von 620-750 nm,
wobei der photosensitive Sensor angepasst ist, um Licht der zumindest zwei unterschiedlichen Wellenlängen zu erfassen, welches nicht durch Blut oder Blutkomponenten absorbiert wurde, und
wobei das Gerät dazu angepasst ist, die An- oder Abwesenheit von Blut durch Vergleich der Intensitäten des Lichts der zumindest zwei unterschiedlichen Wellenlängen zu erkennen, die durch den photosensitiven Sensor erfasst wurden,
wobei mindestens ein Recess (6) in der Kapsel (1) geformt ist, der sich in einer Richtung senkrecht zu einer Längsachse der Kapsel erstreckt und gerundete Kanten aufweist, um die Schluckbarkeit der Kapsel (1) zu verbessern,
wobei die Lichtquelle (3) und der photosensitive Sensor (4) so angeordnet sind, dass diese sich über den mindestens einen Recess (6) gegenüberliegen, und
wobei der mindestens eine Recess (6) einen freien Raum ausbildet, in den die zu untersuchende Substanz/Inhalt fließen kann.

2. Gerät zur Blutungserkennung nach Anspruch 1, wobei die äußere Form der Kapsel (1) eine Pillenform, eine ovale Form, eine Eierform oder eine zylindrische Form mit gerundeten Kanten/Enden ist.

3. Gerät zur Blutungserkennung nach Anspruch 1 oder 2, wobei das mindestens eine Erkennungsmittel (3, 4) dazu angepasst ist, die An- oder Abwesenheit von Blut in einer Substanz/Inhalt durch Messen des spezifischen Absorptionsspektrums der Substanz/Inhalt zu erkennen.

4. Gerät zur Blutungserkennung nach Anspruch 1, wobei die Lichtquelle (3) und der photosensitive Sensor (4) so angeordnet sind, dass das Licht (5), welches durch die Lichtquelle (3) emittiert wird, den Recess (6) passiert und zumindest teilweise durch den photosensitiven Sensor (4) erkannt werden kann.

5. Gerät zur Blutungserkennung nach einem der vorhergehenden Ansprüche, wobei das Gerät zur Blutungserkennung weiter dazu angepasst ist, zwischen verschiedenen Manifestationen von Blut zu unterscheiden, z.B. ob es sich um venöses, arterielles oder geronnenes Blut handelt.

6. Gerät zur Blutungserkennung nach einem der vorhergehenden Ansprüche, weiter mit einer Photo/Video-Kamera, vorzugsweise in Form eines CCD-Sensors, die in der Kapsel (1) vorgesehen ist, zur Aufnahme von Bildern in der Nähe der Kapsel (1).

7. Gerät zur Blutungserkennung nach Anspruch 1, weiter mit einem Übertragungsmittel (9), welches innerhalb der Kapsel (1) vorgesehen ist, um Daten zu senden, die mit den Erkennungsmitteln (3, 4) erkannt werden.

## Revendications

1. Dispositif pour la détection d'hémorragie consistant en une capsule (1) et au moins un moyen de détection (3, 4) prévu dans la capsule (1) pour détecter une présence ou une non présence de sang,
dans lequel la capsule (1) a une forme extérieure et une dimension pour pouvoir être avalée par un patient,
dans lequel les moyens de détection comprennent une source de lumière (3), de préférence au moins une DEL, et un capteur photosensible (4), de préférence au moins une photodiode ou un phototransistor, pour recevoir la lumière émise par la source de lumière (3),
dans lequel la source de lumière (3) est conçue pour émettre alternativement une lumière d'au moins deux longueurs d'onde différentes absorbées à un degré différent par le sang ou des composants du sang, à savoir une lumière dans la plage violette du spectre visible ayant une longueur d'onde dans la plage de 380 à 450 nm et une lumière dans la plage rouge du spectre visible ayant une longueur d'onde dans la plage de 620 à 750 nm,
dans lequel le capteur photosensible est conçu pour détecter la lumière à au moins deux longueurs d'onde qui n'ont pas été absorbées par le sang ou des composants du sang, et
dans lequel le dispositif est conçu pour déterminer une présence ou une non présence de sang en comparant les intensités de la lumière aux dites au moins deux longueurs d'onde différentes détectées par le capteur photosensible,
dans lequel au moins un évidement (6) est formé dans la capsule (1), s'étend dans une direction perpendiculaire à un axe longitudinal de la capsule et comporte des bords arrondis pour améliorer la capacité d'avalement de la capsule (1),
dans lequel la source de lumière (3) et le capteur photosensible (4) sont agencés de sorte qu'ils se fassent mutuellement face par l'intermédiaire dudit au moins un évidement (6), et
dans lequel ledit au moins un évidement (6) forme un espace libre dans lequel une substance/un contenu à examiner peut s'écouler.

2. Dispositif pour la détection d'hémorragie selon la revendication 1, dans lequel la forme extérieure de la capsule (1) est une forme similaire à une pilule, une forme ovale, une forme ovoïde ou une forme cylindrique avec des bords/extrémités arrondis.

3. Dispositif pour la détection d'hémorragie selon la revendication 1 ou 2, dans lequel ledit au moins un moyen de détection (3, 4) est conçu pour détecter une présence ou une non présence de sang dans une substance/un contenu en mesurant les spectres d'absorption spécifiques de la substance/du contenu.

4. Dispositif pour la détection d'hémorragie selon la revendication 1, dans lequel la source de lumière (3) et le capteur photosensible (4) sont agencés de sorte que la lumière (5) émise par la source de lumière (3) traverse l'évidement (6) et puisse être au moins partiellement détectée par le capteur photosensible (4).

5. Dispositif pour la détection d'hémorragie selon l'une quelconque des revendications ci-dessus, dans lequel le dispositif pour la détection d'hémorragie est en outre conçu pour effectuer une différenciation entre différentes manifestations de sang, par exemple si le sang est du sang veineux, artériel, ou coagulé.

6. Dispositif pour la détection d'hémorragie selon l'une quelconque des revendications ci-dessus, comprenant en outre un appareil photographique/une caméra vidéo, de préférence sous la forme d'un capteur CCD, prévu dans la capsule (1) pour capturer des images du voisinage de la capsule (1).

7. Dispositif pour la détection d'hémorragie selon la revendication 1, comprenant en outre des moyens d'envoi (9) prévus à l'intérieur de la capsule (1) pour envoyer les données détectées par les moyens de détection (3, 4).
